# EUROPEAN PATENT APPLICATION

(11) **EP 4 593 032 A1**
(43) Date of publication of application: **30.07.2025**
(21) Application number: 24223654.5
(22) Date of filing: 30.12.2024
(51) Int. Cl.: G16H 40/63

(54) **METHOD FOR DISPLAYING USER INTERFACE, MEDICAL DEVICE, AND NON-TRANSITORY COMPUTER-READABLE MEDIUM**

(30) Priority: 23.01.2024 CN 202410097751
(71) Applicant: GE Precision Healthcare LLC, Waukesha, WI 53188 (US)
(72) Inventor: MA, You, Waukesha, 53188 (US); LIU, Yiwei, Waukesha, 53188 (US); PU, Hui, Waukesha, 53188 (US); LIU, Jia, Waukesha, 53188 (US)
(74) Representative: Fennell, Gareth Charles

(57) **Abstract**

Provided in the present application is a method for displaying a user interface of a medical device, including: displaying a medical information region and a function region on the user interface, the function region including a plurality of icons, and the plurality of icons corresponding to a plurality of functions of the medical device, respectively; automatically performing availability detection on the plurality of functions of the medical device at a current time point; and automatically changing display states of the icons based on results of the availability detection. Further provided in the present application are a medical device and a non-transitory computer-readable medium.

## Description

### TECHNICAL FIELD

The present invention relates to the medical field, and in particular, to a method for displaying a user interface of a medical device, a medical device, and a non-transitory computer-readable medium.

### BACKGROUND

With the progress of medical technology, various medical devices are widely used in patient health care. In order to accommodate different usage needs of users, medical devices are tasked with different functions for different usage scenarios. A patient monitor is one type of medical device. Using a patient monitor, a user may monitor vital signs of a patient in real time and promptly gain knowledge about physiological conditions of the patient to make proper decisions.

With the progress of technology, patient monitors have gradually been tasked with increasingly rich functions. On the one hand, integration has allowed the size and weight of patient monitors to be kept within a reasonable range. However, on the other hand, the limited screen size of patient monitors is tasked with simultaneously displaying a plurality of physiological parameters and a large number of options for users to operate. The compact arrangement of various types of information on the limited screen may easily lead to visual fatigue for users. Moreover, searching through the multitude of available options to find a specific option that currently needs to be operated may become quite cumbersome.

### SUMMARY

The aforementioned defects, deficiencies, and problems are solved herein, and these problems and solutions will be understood through reading and understanding the following description.

Provided in some embodiments of the present application is a method for displaying a user interface of a medical device, comprising: displaying a medical information region and a function region on the user interface, the function region comprising a plurality of icons, and the plurality of icons corresponding to a plurality of functions of the medical device, respectively; automatically performing availability detection on the plurality of functions of the medical device at a current time point; and automatically changing display states of the icons based on results of the availability detection.

Further provided in some embodiments of the present application is a medical apparatus, comprising a processor and a touchscreen. The touchscreen displays the user interface and is touch-operable. The processor is configured to perform the following method for displaying a user interface: displaying a medical information region and a function region on the user interface, the function region comprising a plurality of icons, and the plurality of icons corresponding to a plurality of functions of the medical device, respectively; automatically performing availability detection on the plurality of functions of the medical device at a current time point; and automatically changing display states of the icons based on results of the availability detection.

Further provided in some embodiments of the present application is a non-transitory computer-readable medium, the non-transitory computer-readable medium having a computer program stored thereon, the computer program having at least one code segment, and the at least one code segment being executable by a machine to enable the machine to perform the steps of the following method: displaying a medical information region and a function region on the user interface, the function region comprising a plurality of icons, and the plurality of icons corresponding to a plurality of functions of the medical device, respectively; automatically performing availability detection on the plurality of functions of the medical device at a current time point; and automatically changing display states of the icons based on results of the availability detection.

It should be understood that the brief description above is provided to introduce, in a simplified form, concepts that will be further described in the detailed description. The brief description above is not meant to identify key or essential features of the claimed subject matter. The scope is defined uniquely by the claims that follow the detailed description. Furthermore, the claimed subject matter is not limited to implementations that solve any deficiencies raised above or in any section of the present disclosure.

### BRIEF DESCRIPTION OF THE DRAWINGS

The present application will be better understood by reading the following description of non-limiting embodiments with reference to the accompanying drawings, where:
FIG. 1 is a schematic diagram of a medical device connected to a patient according to some embodiments of the present application;
FIG. 2 is a flowchart of a method for displaying a user interface of a medical device according to some embodiments of the present application;
FIG. 3 is a schematic diagram of a user interface of a medical device according to some embodiments of the present application;
FIG. 4 is a schematic diagram of a user interface after the user interface in FIG. 3 is adaptively adjusted;
FIG. 5 is a schematic diagram of another user interface after the user interface in FIG. 3 is adaptively adjusted;
FIG. 6 is a schematic diagram of a user interface of a medical device according to some other embodiments of the present application;
FIG. 7 is a schematic diagram of a user interface after a collapse icon is operated according to some embodiments of the present application; and
FIG. 8 is a schematic diagram of an EWS display interface according to some embodiments of the present application.

### DETAILED DESCRIPTION

Specific implementations of the present invention will be described below. It should be noted that in the specific description of the implementations, it is impossible to describe all features of the actual implementations of the present invention in detail, for the sake of brief description. It should be understood that in the actual implementation process of any implementation, just as in the process of any one engineering project or design project, a variety of specific decisions are often made to achieve specific goals of the developer and to meet system-related or business-related constraints, which may also vary from one implementation to another. Furthermore, it should also be understood that although efforts made in such development processes may be complex and tedious, for those of ordinary skill in the art related to the content disclosed in the present invention, some design, manufacture, or production changes made on the basis of the technical content disclosed in the present disclosure are only common technical means, and should not be construed as the content of the present disclosure being insufficient.

Unless otherwise defined, the technical or scientific terms used in the claims and the description should be as they are usually understood by those possessing ordinary skill in the technical field to which they belong. "First", "second", and similar words used in the present invention and the claims do not denote any order, quantity, or importance, but are merely intended to distinguish between different constituents. The terms "one" or "a/an" and similar terms do not express a limitation of quantity, but rather that at least one is present. The terms "include" or "comprise" and similar words indicate that an element or object preceding the terms "include" or "comprise" encompasses elements or objects and equivalent elements thereof listed after the terms "include" or "comprise", and do not exclude other elements or objects. The terms "connect" or "link" and similar words are not limited to physical or mechanical connections, and are not limited to direct or indirect connections.

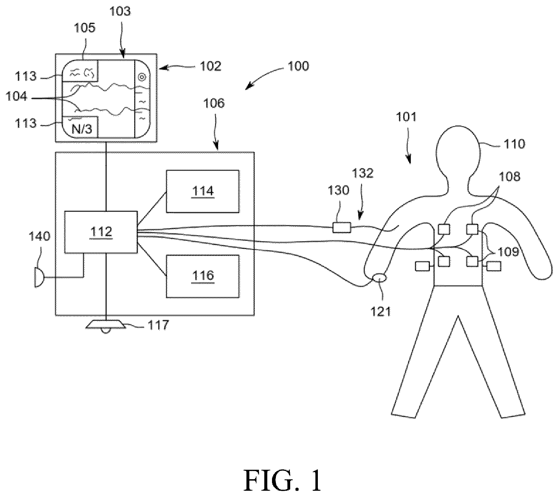

Referring to FIG. 1 first, FIG. 1 shows a schematic diagram of a medical device 100 connected to a patient 110 according to some embodiments of the present application.

The medical device 100 shown in FIG. 1 may be used to monitor various vital signs or parameters of the patient 110 operably connected to the medical device 100. The medical device may be a monitoring or diagnostic apparatus and system of a monitoring apparatus of any suitable type, e.g., a patient monitor. The medical device 100 includes a display 102 of any suitable type having a user interface 103, e.g., a touchscreen display. The touchscreen allows the user interface 103 to include a region for human-computer interaction, e.g., a virtual key. The user interface 103 includes a medical information region 104. The medical information region 104 is used to display a monitoring data signal of the patient 110 connected to the medical device 100. Furthermore, the user interface 103 also includes a function region 105 for performing operations such as human-computer interaction. In one example, the function region 105 may include one continuous region. In another example, the function region may include, as shown in FIG. 1, a plurality of discrete function regions 113. Moreover, in some examples, in addition to the function region 105, a user input device may include a separate component connected to the medical device 100, e.g., a keyboard (not shown) or a mouse (not shown).

In the embodiment of FIG. 1, the medical device 100 may include a host 106. The medical device 100 further has sensors 109 of types such as an impedance respiration sensor/a respiration sensor. FIG. 1 further shows a connection mode 101 between the sensors and the patient. Specifically, the sensors 109 are operably connected between the host 106 and the patient 110 by any suitable means (e.g., a cable), so as to monitor various physiological parameters of the patient 110. In addition to the sensors 109, the medical device 100 may employ other types of sensors 108 for monitoring other parameters or statistics of the patient 110, such as a blood oxygen sensor 121 and/or an invasive pressure catheter 130 and an invasive pressure transducer 132 to measure a blood flow and a blood pressure of the patient 110. In an optional embodiment, the physiological parameters described above may be compared with data obtained from the sensors 109.

The host 106 includes a processor 112, which is operably connected to the sensors 108 and 109 so as to receive and process data from the sensors 108 and 109 regarding various vital signs, statistics, or parameters of somatic functions of the patient 110. The host is related to a respiratory function in the exemplary embodiment of FIG. 1. It may be understood that another somatic function or system is also considered as being within the scope of the present invention. The parameter data may then be processed from the processor 112. Processed data may be transmitted to the display 102 for presentation in a specified manner on the user interface 103 of the display 102, thereby facilitating viewing by a user. In an optional embodiment, the processor 112 may be operably connected to a network (not shown), by means of, e.g., a wired or wireless connection. Among other examples, a television, a health monitor, a cell phone or similar apparatus, a laptop computer, a portable electronic product, etc., are included.

The medical device 100 also includes a memory 114 in the form of a suitable computer-readable electronic storage medium, e.g., a RAM module. The memory 114 is connected to the processor 112, so as to store the data from the sensors 108 and 109 and the processed data from the processor 112. The memory 114 is further adapted to store suitable algorithms for calling by the processor 112. The medical device 100 may further include a power supply 116. The power supply 116 may provide power to the processor 112 and the medical device 100 as a whole in some usage scenarios.

In an optional embodiment, the medical device 100 may further include input devices such as an audio speaker 117 and a microphone 140. The described input devices enable the medical device 100 to provide and receive audible indications of various operational characteristics of the medical device 100.

In the example of FIG. 1, the display 102 and the host 106 are configured to be separately arranged. However, in an optional example, the display and the host may be integrally arranged. An integrated arrangement further helps reduce the volume of the medical device 100, and makes the device more portable. Moreover, it should be understood that the schematic diagram 100 merely provides an exemplary illustration using a patient monitor as an example. In a further example, the medical device 100 may further be of another type, e.g., any medical device having a user interface that includes both a medical information region and a function region. For example, the medical device 100 may be any device such as a patient monitor, an electrocardiograph, an anesthesia machine, or a ventilator. In such a medical device, a large amount of listed medical information (e.g., the physiological parameters of the patient) and many icons (e.g., virtual keys, etc.) in the function region cause the user interface to contain a huge amount of information. A user may encounter many inconveniences when observing and selecting a function to be operated. For example, attempts to list a large number of function keys in a limited user interface size often require compromises in the sizes and layout spacings of the function keys. Compact and small-sized function keys bring about inconveniences in selection and make it easy to perform an erroneous operation during operation. At least in view of this, improvements are provided in some embodiments of the present application.

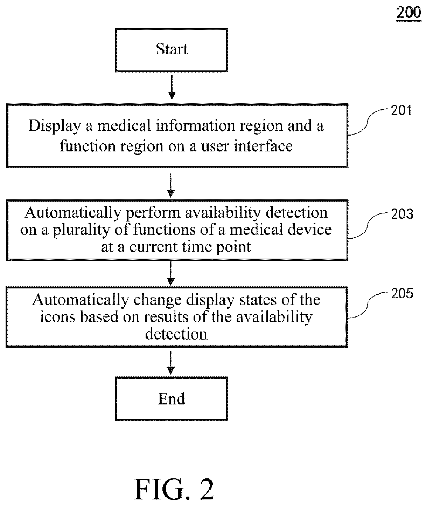

Referring to FIG. 2, FIG. 2 shows a method 200 for displaying a user interface of a medical device according to some embodiments of the present application. The method may be implemented by a processor, e.g., the processor of the medical device in FIG. 1 or any of the embodiments of the present application.

Step 201: displaying a medical information region and a function region on the user interface. The function region includes a plurality of icons, and the plurality of icons correspond to a plurality of functions of the medical device, respectively.

Step 203: automatically performing availability detection on the plurality of functions of the medical device at a current time point.

Step 205: automatically changing display states of the icons based on results of the availability detection.

Such a configuration manner may ensure that, when faced with medical information and operable function information (e.g., function icons) arranged on a user interface having a limited size, a user may exclude useless information as quickly and accurately as possible, and accurately find a required function icon. Specifically, in a solution of the present application, the processor may automatically monitor whether function icons representing different functions in the current user interface are available at this time point. Based on the availability, the processor may adaptively adjust an icon display state in the user interface. For example, the processor may highlight an available icon, and hide an unavailable icon from display. In this way, limited space may be used as much as possible for presenting useful information. For a compact medical device, such a manner may simplify the display of the user interface as much as possible, thereby improving the operation efficiency of the user.

In the embodiments of the present application, the availability of a function may be understood as the possibility that the function may be executed (used) or needs to be executed in a current environment. That is, the processor may perform availability detection on the functions displayed on the user interface. The availability detection may be performed by various means, which will be exemplified below.

In some embodiments, the availability detection may include detecting whether the medical device is currently configured with the functions. In a conventional user interface display mode, the functions on and layout of a user interface are set. That is, after a user opens a certain user interface, regardless of whether functions corresponding to icons in a function region on the user interface are available, the icons are all laid out on the user interface. Although in some examples, icons corresponding to unavailable functions may be displayed with special visual effects (e.g., in gray), neither the user interface space occupied nor the workload of the user when selecting an icon may be reduced. In this example of the present application, it is detected whether the functions are currently configured. That is, a function not configured is determined to be an unavailable function, and accordingly, a display state of the corresponding icon is changed (e.g., hidden), thereby freeing more space on the user interface for other purposes. It may be understood that whether the functions are configured may be detected by various means. In one example, when a function is provided by an accessory device, the processor may determine whether the medical device is connected to the accessory device. If the accessory device is not detected, it is directly determined that the function is not configured, and the function is unavailable. In this case, the corresponding icon may be directly hidden. If it is detected that the accessory device is properly connected, it is directly determined that the function has been configured, and the function is in an available state. In this case, the corresponding icon may not be hidden. In another example, the functions may be provided by software. The type of an accessory device may be an accessory device of the medical device, e.g., a sensor, etc. An accessory device, e.g., a printer, other than an accessory device of the medical device may also be used.

In some embodiments, the availability detection may include detecting whether the medical device is currently executing the functions. For example, a function being executed by the medical device may be determined to be an available function. In this case, according to the urgency or importance of the function being executed, a display state of an icon corresponding to the function being executed may be automatically changed for the purpose of making the icon more prominent and easier to operate. For example, the icon of the function being executed may be enlarged (an extra area for the enlargement may be allocated from areas of other hidden icons). For a function not being executed, it is considered that the function is unavailable (or does not need to be executed). The size of an icon corresponding to the function not being executed may be reduced or even hidden, so that saved space may be allocated to the icons corresponding to the available functions.

In still other embodiments, the availability detection may include detecting whether the possibility of executing the functions exists in a current usage environment of the medical device. The same type of medical device may face different usage environments. Taking a patient monitor as an example, the patient monitor may be used in a general ward, an intensive care unit, an operating room, etc. A patient monitor in a general ward is typically used to perform routine monitoring of physiological parameters, whereas a patient monitor in an operating room is adapted to monitor physiological parameters related to anesthesia, etc. (e.g., a bispectral index). Accordingly, a function of monitoring the physiological parameters related to anesthesia is less likely to be used in the general ward. In this case, a change in the display of icons corresponding to anesthesia-related functions may be performed, for example, the icons may be hidden. The same rule applies to instruments other than patient monitors. Examples are not exhaustively enumerated herein.

Moreover, in some examples, the means of availability detection may be one or a combination of a plurality of the means described in the foregoing embodiments. The combination of a plurality of the means may further improve the display efficiency of the icons on the user interface, thereby improving the operation efficiency of the user.

It may be understood that a change to the display state of an icon described in the examples of the present application includes at least a change to the space occupied by the icon on the user interface. Only in this way is it possible to solve the problem that the icons and the medical information on the user interface are displayed in too compact a manner and display effects matching the icons cannot be allocated.

In some examples, automatically changing display states of the icons based on results of the availability detection includes: in response to detecting at least one unavailable function, automatically hiding an icon in the function region corresponding to the at least one unavailable function. An unavailable function generally means that the function has a lower priority at the current time point. At this point, it is extremely unlikely that the icon corresponding to the function is operated. In this case, it becomes unnecessary to display the icon on the user interface. In an example of the present application, an icon corresponding to an unavailable function may be automatically hidden. On the one hand, the hidden icon does not need to be filtered by the user as a candidate in the function region on the user interface. On the other hand, the space saved by hiding the icon may be utilized by an icon with a higher priority or more useful medical information, thereby further improving the usage efficiency of the user.

The space saved by hiding the icon can be used to perform updated display of an unhidden icon in the function region. An exemplary description is provided below.

In an example, the foregoing updated display may include adding a new icon. In practical usage scenarios, a limited user interface often cannot display all available functions of a medical device. In this case, a conventional practice is to configure the user interface so that the user interface may be manually switched to another, and to arrange, on the other user interface, icons that cannot be simultaneously displayed. This makes operation troublesome. In the foregoing example, a new icon (an icon corresponding to an available function) may be adaptively added to the current user interface, so as to reduce the probability that the user needs to switch the user interface.

Moreover, in a preferred example, an arrangement manner of the icons in the function region may be as follows: the icons are sorted based on priorities of the corresponding functions that are automatically determined by the processor. In this manner, the usage efficiency of the user may be further improved in conjunction with the foregoing embodiments of the present application. Specifically, the newly added icon will be an icon corresponding to a high-priority function determined by the processor. In this case, since the function of the added icon is more easily used by the user, the function becomes more user-friendly and more suitable for actual usage scenarios.

In a further example, the foregoing updated display may further include enlarging the unhidden icon. For example, when no new icon needs to be added, or when a function corresponding to an icon already displayed on the user interface has a high priority (e.g., an emergency such as an alarm), at least some unhidden icons may be enlarged for convenient observation and selection by the user.

In some examples, the updated display may further include a combination of any of the foregoing examples, and details are not described again here.

It is recognized by the inventor that a use state of a medical device may vary from time to time. For example, a function that is initially unavailable may become available even during a single use. This may cause inconvenience to the user if an icon corresponding to the function has been hidden. The inventor has made improvements regarding this problem. In some embodiments, automatically changing display states of the icons based on results of the availability detection further includes: performing availability detection on the function corresponding to the hidden icon in real time; and in response to availability of the corresponding function being resumed, resuming display of the hidden icon.

Such an implementation ensures that the processor may instantly determine whether the function corresponding to the hidden icon has changed. A patient monitor is still used as an example for description. In one example, when the patient monitor is not initially connected to a certain sensor, a function icon corresponding to the sensor may be hidden on the user interface. When the user needs to use the sensor, the user simply needs to connect the sensor to the patient monitor. At this point, the icon corresponding to the sensor adaptively appears on the user interface according to the embodiments of the present application. It may be understood that, once the icon is added to the user interface, if there is no extra space at this point, the sizes of all icons on the user interface may be adjusted, and operations such as collapsing may be performed on low-priority icons based on priorities. Examples are not exhaustively enumerated herein.

In actual usage scenarios, a result of automatic determination by the processor may have a certain deviation, for example, an icon that the user actually does not want to hide may be hidden for certain reasons. Moreover, in some other scenarios, the size of the user interface does not allow for simultaneous presentation of the icons of all functions in the function region. In view of the foregoing cases, in some embodiments of the present application, the function region further includes a collapse icon, and the collapse icon is operable to display a user interface including all hidden icons.

With such a configuration manner, even if some icons are hidden after the availability detection, the user may still open the user interface including all the hidden icons by quickly operating the collapse icon (e.g., by tapping with a finger), and then select therefrom an icon that actually needs to be operated. Moreover, if some icons cannot be displayed on the user interface due to a size problem of the function region, this problem may be effectively solved in the present embodiments.

As described above, some medical devices may have many functions, and icons corresponding to these functions cannot be displayed simultaneously on the same user interface. In addition to providing a collapse icon to solve the foregoing problem, some embodiments of the present application further provide an optimization solution. The processor may further adaptively prioritize the plurality of icons displayed in the function region. That is, in a case where size is limited, the processor may select icons to be displayed and the arrangement order of these icons based on priorities. For example, some high-priority icons are selected for display, and/or priorities of these icons are further sorted according to priorities. With such a configuration manner, as many icons urgently needing to be used by the user may be displayed in the function region as possible, thereby further reducing the complexity of user operations.

It may be understood that priorities may be determined in various ways. In some embodiments, the priorities may be determined according to degrees of importance of the functions. For example, functions related to detection of vital signs (heart rate, oxygen saturation, etc.) of the current patient are considered to be high-priority, whereas functions related to parameter adjustment (e.g., screen brightness, battery level, etc.) of instruments and devices are automatically considered to be low-priority. In a further embodiment, the priorities may further be adaptively adjusted based on different usage scenarios. For example, the processor determines a current usage scenario of the medical device, and further determines high-priority functions in this scenario based on the current usage scenario. Taking a patient monitor as an example, in an operating room, functions related to monitoring of an anesthetic state will have high priorities. In a ward round mode, functions such as patient parameter recording and monitoring of general signs will have higher priorities. The foregoing will not be further enumerated.

According to the present application, the display state of the icons may be automatically changed based on the results of the availability detection. In addition to operations such as adaptively hiding an icon corresponding to a function whose detection result is displayed as "unavailable" as described in the foregoing embodiments, the following may be further included: in response to detecting at least one available function or at least one function being executed, automatically highlighting an icon in the function region corresponding to the at least one available function or the at least one function being executed. Such an implementation improves the working efficiency of the user from another perspective. That is, when a plurality of icons are present in the function region, the processor may automatically determine, based on a current usage environment, whether a certain function or a plurality of functions are available or being executed. If a certain function or a plurality of functions are available or being executed, the processor adaptively highlights an icon corresponding to the one function or the plurality of functions, thereby helping the user more intuitively observe and operate the icon to be operated.

It may be understood that the highlighting may be performed in various manners. A detailed description will be provided below.

In some examples, the highlighting may include enlarging the icon for display. In scenarios outside the embodiments of the present application, it is difficult to achieve enlargement for display. A main reason is that a display interface without an adaptive adjustment function is usually occupied by various icons and medical information, and there is no space to enlarge a certain icon or a plurality of icons for display. In the examples of the present application, hiding an icon corresponding to an unavailable function may provide extra screen space. In this case, the extra screen space may be used for the enlarged display of an icon that needs to be highlighted.

In some examples, the highlighting may include changing the color of the icon. It is recognized by the inventor that, even if the icon corresponding to the unavailable function is hidden according to the foregoing examples in the present application, the adaptively adjusted screen may still include a large number of icons of available functions. In this case, the color of an icon may be changed so that the icon that needs to be highlighted is displayed in a more noticeable way. For example, the colors of some icons with higher priorities or higher degrees of urgency may be changed. For example, the icons may be changed to colors, such as eye-catching red or yellow, different from the colors of other icons. In this way, even if being faced with a user interface upon which a large number of icons are arranged, the user can notice, in a short time, an icon of which the color has been changed.

In some examples, the highlighting may further include floating the icon over the medical information region for display. Floating a displayed icon will provide convenience in a number of aspects. On the one hand, an icon displayed by floating is more easily found by the user than an icon arranged in a fixed manner. On the other hand, the floating display makes it easier to adjust the size of the icon. For example, in an example, the size of an icon displayed by floating may be enlarged. Since the icon is floating, the enlargement of the icon does not occupy display space for other icons or medical information. In particular, when an available function is being used, the processor may display, in response to a certain function being used, an icon corresponding to the available function by floating. The space saved by the floating display may be used for the enlarged display of medical information corresponding to the foregoing function. In this way, on the one hand, observation of the medical information region by the user is more convenient, and on the other hand, a floating position of an icon displayed by floating may be moved to an appropriate position, which does not affect observation of medical information and may facilitate operation.

In a further embodiment, the highlighting may include a combination of any of the manners above. For example, an icon is enlarged while the color thereof is changed, or an icon is floated while undergoing appropriate enlargement and the color thereof is changed (including a change in transparency), etc. Examples are not exhaustively enumerated herein.

The method for displaying a user interface in the foregoing description of the present application is described in detail below with reference to the accompanying drawings. It should be noted that, in an example of the present application, an interface of a patient monitor is taken as an example for detailed description, but those skilled in the art may apply the teachings of the present disclosure to other medical devices facing the same problem, so as to improve user operation efficiency.

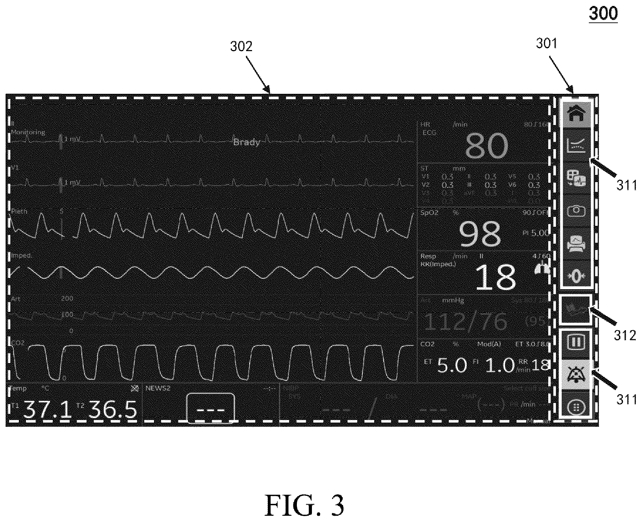

Referring to FIG. 3, FIG. 3 shows a schematic diagram of a user interface 300 of a medical device according to some embodiments of the present application. As shown in FIG. 3, the user interface 300 may include a medical information region 302 and a function region 301. The medical information region 302 displays various desired physiological parameters of a patient for reference by a user (e.g., a doctor). The function region 301 includes a plurality of icons operable by the user. The layout and functions of the icons may vary according to different medical devices or user settings. In the present application, availability detection may be performed on the functions corresponding to these icons, as described in any of the corresponding embodiments above in the present application. Through the availability detection, icons 311 corresponding to available functions and an icon 312 corresponding to an unavailable function in the function region 301 may be determined. The manner of availability detection may be the manner described in the foregoing embodiments of the present application, and details are not described herein again. In the example of FIG. 3, the icon 312 may represent non-invasive blood pressure measurement. The manner of determining that the function is unavailable may be based on the fact that a non-invasive blood pressure measurement apparatus is not connected, or that invasive blood pressure measurement is being performed, etc. It may be clearly seen from the example of FIG. 3 that, although the function corresponding to the icon 312 is determined to be unavailable, the layout of the user interface 300 as a whole does not change. Icons corresponding to unavailable functions remain fixed on the user interface 300. Icons that are not actually needed or cannot be used not only occupy extra space, but also interfere with determination and decision-making by the user during actual operation.

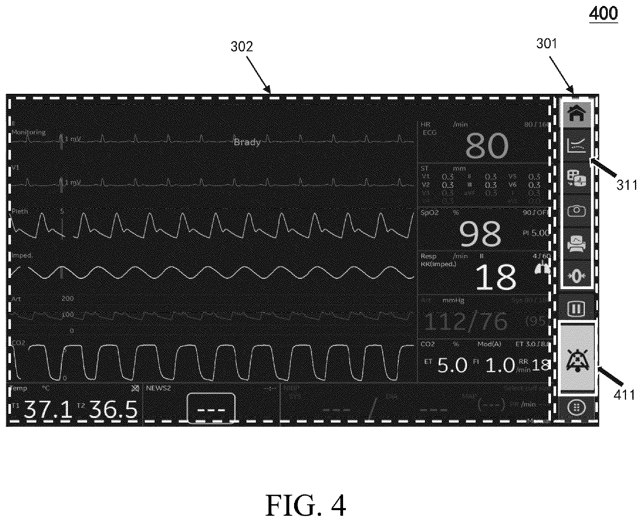

The adaptive user interface adjustment manner of the present application solves the foregoing problem. Continuing to refer to FIG. 4, FIG. 4 shows a schematic diagram of a user interface 400 after the user interface in FIG. 3 is adaptively adjusted. Details of other similar parts are not described again here. The adaptive adjustment manner of the present application will be described in detail with reference to FIG. 3 and FIG. 4. In an example of the present application, after the availability detection, in response to detecting at least one unavailable function (e.g., the non-invasive blood pressure measurement function corresponding to the icon 312 in FIG. 3), the icon 312 in the function region corresponding to the at least one unavailable function may be automatically hidden. It may be seen from FIG. 4 that the icon 312 initially present in the user interface 300 of FIG. 3 has been hidden, and the space initially occupied by this icon has been freed for further use.

It should be noted that, in the foregoing embodiments, the function region is a continuous complete region, but in an optional embodiment, the function region may be dispersedly distributed at multiple positions of the user interface (e.g., the manner illustrated in FIG. 1).

Moreover, as in the embodiments described above in the present application, in the manner of changing the display state of the images based on the results of the availability detection, an icon corresponding to a detected available function may be further highlighted, in addition to hiding the icon corresponding to the unavailable function (e.g., the icon 311 of FIG. 3). A description is provided with reference to FIG. 3 and FIG. 4. The icon 311 corresponding to an unavailable function in FIG. 3 is hidden in FIG. 4, and accordingly, an icon of an available function, e.g., an icon 411 in FIG. 4, may be enlarged. In this way, the icon 411 is more easily observed and more easily operated (e.g., tapped) by the user. In this embodiment, hiding the icon of an unavailable function and highlighting the icon of an available function cooperate with each other. On the one hand, the hiding operation provides extra screen space. On the other hand, the extra screen space is further fully utilized by the icon of an available function.

It may be understood that the embodiment of FIG. 4 shows a case in which only one icon 411 (e.g., an alarm icon) is highlighted. In a further example, alternatively, a plurality of icons may be highlighted. Examples are not exhaustively enumerated herein.

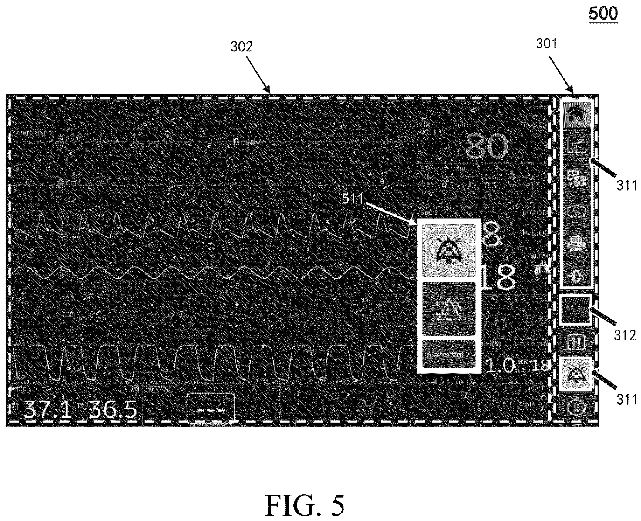

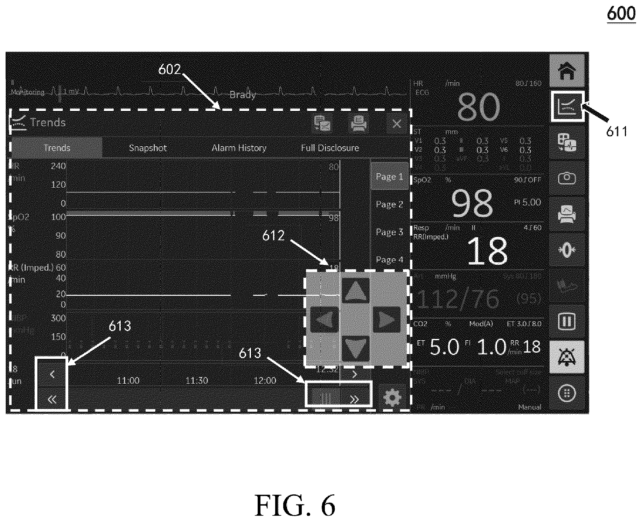

In addition to the foregoing highlighting manners, in some additional embodiments, the highlighting may further include floating the icon over the medical information region for display. With such a configuration manner, on the one hand, a case in which no occupiable screen space for highlighting does not exist, and on the other hand, the highlighting effect is more obvious. It may be understood that a highlighted icon may correspond to an available function not in use, but in a preferred example, a highlighted icon corresponds mainly to a function being used. An exemplary description will be provided below with reference to FIG. 5 and FIG. 6.

Referring first to FIG. 5, FIG. 5 shows a schematic diagram of another user interface 500 after the user interface in FIG. 3 is adaptively adjusted. An icon 511 is displayed floating over the medical information region 302. In this embodiment, the processor detects that an alarm is occurring, i.e., a function for adjusting the alarm may be used at this point. Displaying the corresponding icon 511 by floating, on the one hand, facilitates timely discovery by the user, and on the other hand, helps the user perform an operation. Compared to the alarm icon in the function region 301, the icon 511 may be configured with a larger size and more functions (which may be configured, sequentially from top to bottom, as, e.g., mute alarm, jump to alarm details, adjust alarm volume, etc.,). It should be noted that, in the example of FIG. 5, the icon 312 corresponding to an unavailable function is not hidden. However, the example of FIG. 5 may be combined with the adaptive user interface adjustment manner of any of the embodiments of the present application under the teachings of the present disclosure. For example, the adaptive highlighting of the icon corresponding to an available function in FIG. 5 may be used in conjunction with the adaptive hiding of the icon corresponding to an unavailable function in FIG. 4. Examples are not exhaustively enumerated herein.

Referring to FIG. 6 next, FIG. 6 is a schematic diagram of a user interface 600 of a medical device according to some other embodiments of the present application. FIG. 6 shows another highlighting manner, in addition to the highlighting manner of FIG. 5 (highlighting an icon that may be used). Reference is made to FIG. 3 and FIG. 6. In one scenario, by means of operating an icon 613, the medical information region of the user interface may jump to a medical information region 602 of FIG. 6 from another (e.g., the medical information region 302 of FIG. 3). The medical information region 602 of FIG. 6 is described using a physiological parameter change trend as an example. In a non-adaptive user interface, the user may adjust function region icons 613 below a physiological parameter trend line to select a particular time period, a data start time, a data stop time, etc., so as to obtain a desired physiological parameter change trend. It is easy to see that the dispersedly distributed icons above are small in size and difficult to operate. In the embodiment of FIG. 6, after determining a function being used, the processor may generate an icon 612 corresponding to the function, and display the icon by floating. It may be seen from FIG. 6 that the icon 612 has a larger size and is easier to operate. In particular, the adaptively adjusted icon 612 may be more conducive to use by the user than the conventional icon 613.

In addition to the enlarged display, the floating display, etc., for the icon corresponding to an available function, the color of the icon may also be changed for highlighting. For example, the function regions in FIG. 3 to FIG. 6 each have color-changed icons. Such a color-changed icon will be easier to quickly pick out from a large number of icons than the icon corresponding to an unavailable function and/or an icon corresponding to an available but low-priority function.

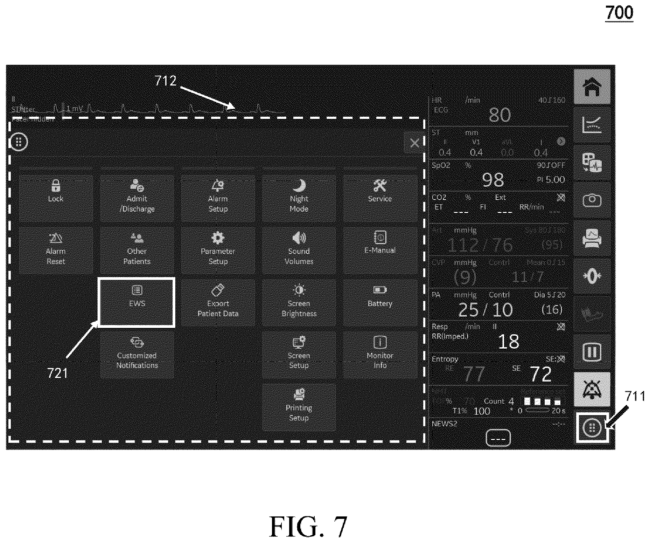

In some examples, the function region further includes a collapse icon, and the collapse icon is operable to display a user interface including all hidden icons. An exemplary description is provided with reference to FIG. 7. A user interface 700 of FIG. 7 illustrates a function region 712 after a collapse icon 711 is operated. The function region 712 includes a large number of function icons, and therefore all hidden icons may be displayed. In this way, even if the processor performs an erroneous operation during an adaptive adjustment process, a hidden icon may be quickly accessed by means of opening the function region 712.

It should be noted that reference may be made to the foregoing embodiments in the present specification for which icons of all the icons are displayed and which are collapsed in the user interface 700, e.g., an icon corresponding to an unavailable function being automatically collapsed and hidden. Alternatively, icons of available functions are sorted based on various factors, such as priorities, degrees of importance, and possibilities of being operated in a current usage environment, and high-priority icons are displayed. The present embodiments may also be combined with the foregoing embodiments of hiding an icon. For example, once the processor hides the icon corresponding to an unavailable function, an icon initially in a collapsed state may be sequentially filled into a non-collapsed function region.

In the embodiments of the present application, availability detection may be performed on a plurality of functions, depending on the type of a medical device. For example, when the medical device is a patient monitor or another device for monitoring vital signs, one of the corresponding functions may include an early warning score (EWS) function. Accordingly, the plurality of icons described above may include an EWS icon, and the EWS icon is operable to display an EWS interface. EWS is a physiological scoring system that is widely used in the medical field. The system may quickly and accurately identify patients at high risk of clinical deterioration based on quickly acquired bedside indicators such as heart rate, respiratory rate, systolic blood pressure, and blood oxygen saturation (SpO2). Now, various EWSs have been developed for early identification of clinical risks. In a usage scenario, the EWS quickly and accurately identifies patients at high risk of clinical deterioration based on a plurality of indicators such as heart rate, respiratory rate, systolic blood pressure, and blood oxygen saturation. In the present application, the type of the foregoing EWS may be any type in the field of patient monitoring, e.g., NEWS, MEWS, etc. The present application sets no limitation thereon. As shown in FIG. 7, an EWS icon 721 is arranged in the function region 712. Alternatively, the EWS icon may be displayed in any other function region. Once the EWS icon is operated, an EWS interface may be displayed for reference by the user.

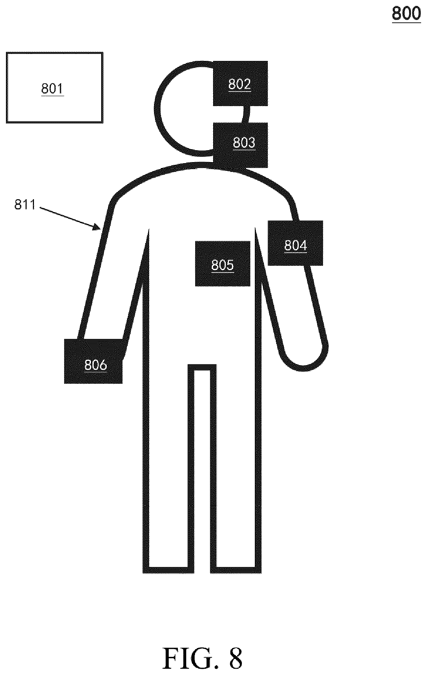

In a preferred example, the EWS interface includes a plurality of score windows for physiological parameters and one human body icon. The plurality of score windows have associations with different parts of the human body, respectively, and are mapped to different parts of the human body icon based on the associations. In this way, the user may not only observe a total EWS score and each sub-score, but also quickly understand clinical parts and clinical meanings corresponding to the sub-scores. An exemplary description is provided below with reference to FIG. 8.

FIG. 8 is a schematic diagram of an EWS display interface 800 according to some embodiments of the present application. The EWS display interface includes a total score window 801 and various sub-score windows 802 to 806. Unlike a conventional EWS display mode, the plurality of sub-score windows 802 to 806 are dispersedly distributed on various parts of the human body icon 811. In addition, the sub-score windows 802 to 806 have associations with different parts of the human body, and are mapped to different parts of the human body icon 811 based on the associations. For example, the sub-score window 802 is mapped to the head of the human head icon 811, and may represent a consciousness score. The sub-score window 803 is mapped to the oronasal portion of the human body icon 811, and may represent a respiratory rate score. The sub-score window 804 is mapped to a brachial portion of the human body icon 811, and may represent a systolic blood pressure score. The sub-score window 805 is mapped to the heart portion of the human body icon 811, and may represent a heart rate score. The sub-score window 806 is mapped to a fingertip of the human body icon 811, and may represent a blood oxygen saturation score.

The above description is merely exemplary, and the EWS may further include other types of sub-scores, which may also be mapped under the foregoing teachings of the present disclosure. Examples are not exhaustively enumerated herein.

In addition to position mapping, the foregoing sub-score windows may provide other functions, e.g., functions similar to icons. When a sub-score window is tapped or otherwise operated, a user interface may be used to display specific information of the sub-score. Moreover, the total score window 801 and the sub-score windows 802 to 806 may be configured in different colors based on the score values. For example, a low score is green, a medium score is orange, and a high score is red. In this way, the user may intuitively and quickly learn about the current criticality of the patient from the interface 800. In addition, by means of the sub-score windows, the user may quickly know which part or parts have an excessively high score, thereby enabling corresponding measures to be taken promptly.

Some embodiments of the present application further provide a non-transitory computer-readable medium, where the non-transitory computer-readable medium has a computer program stored thereon, the computer program has at least one code segment, and the at least one code segment is executable by a machine so as to enable the machine to perform the steps of the method described in any one of the above embodiments of the present application.

Provided in some embodiments of the present application is a medical device, including a processor and a touchscreen. The processor is configured to perform the method for displaying a user interface as described in any of the embodiments above in the present application. The touchscreen displays the user interface and is touch-operable.

It may be understood that, for other components of the medical device, reference may also be made to the components described in any of the embodiments above (e.g., the embodiment of FIG. 1), and details are not described again here.

Moreover, in some of the embodiments above, the medical device is described using a patient monitor as an example. However, the present application is not limited thereto. In some embodiments, the medical apparatus includes at least one of a patient monitor, an electrocardiograph, an anesthesia machine, and a ventilator.

Correspondingly, the present disclosure may be implemented by means of hardware, software, or a combination of hardware and software. The present disclosure may be implemented in at least one computer system in a centralized manner, or implemented in a distributed manner; and in the distributed manner, different elements are distributed on a plurality of interconnected computer systems. Any type of computer system or other apparatus suitable for implementing the methods described herein is considered to be appropriate.

Various embodiments may also be embedded in a computer program product, which includes all features capable of implementing the methods described herein, and the computer program product is capable of executing these methods when loaded into a computer system. The computer program in this context means any expression in any language, code, or symbol of an instruction set intended to enable a system having information processing capabilities to execute a specific function directly or after any or both of the following: a) conversion to another language, code, or symbol; and b) replication in different material forms.

The purpose of providing the above specific embodiments is to facilitate understanding of the content disclosed in the present invention more thoroughly and comprehensively, but the present invention is not limited to these specific embodiments. Those skilled in the art should understand that various modifications, equivalent replacements, and changes can also be made to the present invention and should be included in the scope of protection of the present invention as long as these changes do not depart from the spirit of the present invention.

## Claims

1. A method for displaying a user interface of a medical device, comprising:
displaying a medical information region and a function region on the user interface,
wherein the function region comprises a plurality of icons, and the plurality of icons correspond to a plurality of functions of the medical device, respectively;
automatically performing availability detection on the plurality of functions of the medical device at a current time point; and
automatically changing display states of the icons based on results of the availability detection.

2. The method according to claim 1, wherein the availability detection comprises at least one of the following:
detecting whether the medical device is currently configured with the functions;
detecting whether the medical device is currently executing the functions; and
detecting whether the possibility of executing the functions exists in a current usage environment of the medical device.

3. The method according to claim 1, wherein automatically changing display states of the icons based on results of the availability detection comprises:
in response to detecting at least one unavailable function, automatically hiding an icon in the function region corresponding to the at least one unavailable function.

4. The method according to claim 3, wherein automatically changing display states of the icons based on results of the availability detection further comprises:
performing availability detection on the function corresponding to the hidden icon in real time; and
in response to availability of the corresponding function being resumed, resuming display of the hidden icon.

5. The method according to claim 3, wherein the function region further comprises a collapse icon, and the collapse icon is operable to display a user interface comprising all hidden icons.

6. The method according to claim 3, wherein automatically changing a display state of the icons based on results of the availability detection further comprises:
performing updated display of an unhidden icon in the function region, wherein the updated display comprises at least one of adding a new icon and enlarging the unhidden icon.

7. The method according to claim 1, wherein automatically changing display states of the icons based on results of the availability detection comprises:
in response to detecting at least one available function or at least one function being executed, automatically highlighting an icon in the function region corresponding to the at least one available function or the at least one function being executed.

8. The method according to claim 7, wherein the highlighting comprises at least one of the following:
enlarging the icon for display;
changing the color of the icon; and
floating the icon over the medical information region for display.

9. The method according to claim 1, wherein the plurality of icons comprise an early warning score (EWS) icon, and the EWS icon is operable to display an EWS interface.

10. The method according to claim 9, wherein the EWS interface comprises a plurality of score windows for physiological parameters, and one human body icon; and the plurality of score windows have associations with different parts of the human body, respectively, and are mapped to different parts of the human body icon based on the associations.

11. A medical apparatus, comprising:
a processor, the processor being configured to perform the method for displaying a user interface according to any one of claims 1 to 10; and
a touchscreen, the touchscreen displaying the user interface and being touch-operable.

12. The medical apparatus according to claim 11, the medical apparatus comprising at least one of a patient monitor, an electrocardiograph, an anesthesia machine, and a ventilator.

13. A non-transitory computer-readable medium, the non-transitory computer-readable medium having a computer program stored thereon, the computer program having at least one code segment, and the at least one code segment being executable by a machine to enable the machine to perform the steps of the method according to any one of claims 1 to 10.
